# EUROPÄISCHE PATENTSCHRIFT

(11) **EP 0 835 860 B1**
(45) Veröffentlichungstag und Bekanntmachung des Hinweises auf die Patenterteilung: **13.06.2001**
(21) Anmeldenummer: 97117010.5
(22) Anmeldetag: 01.10.1997
(51) Int. Cl.: C07C 67/08, C07C 69/60, C07C 69/36, C07C 29/70, B01D 53/26

(54) **Verfahren zur Herstellung von Estern oder Alkoholaten unter Abtrennung von Wasser**
Process for the preparation of esters or alcoholates by water separation
Procédé de préparation d'esters ou d'alcoolats au moyen de séparation de l'eau

(30) Priorität: 14.10.1996 DE 19642326; 08.11.1996 DE 19646113
(43) Veröffentlichungstag der Anmeldung: 15.04.1998
(73) Patentinhaber: BAYER AG, 51368 Leverkusen (DE)
(72) Erfinder: Gröschl, Andreas, Dr., 51375 Leverkusen (DE); Winkler, Adolf, Dr., 51373 Leverkusen (DE); Bremen, Josef, Dr., 51379 Leverkusen (DE)

(56) Entgegenhaltungen:
- EP-A- 0 476 370
- EP-A- 0 691 324
- DE-A- 4 019 170

## Beschreibung

Die vorliegende Erfindung betrifft ein Verfahren zur Herstellung von Estern oder Alkoholaten unter Abtrennung von Wasser aus Reaktionsgemischen zur Veresterung von Säuren bzw. Säureanhydriden mit Alkoholen oder aus Reaktionsgemischen zur Herstellung von Alkoholaten aus wässrigen Alkalimetallhydroxid-Lösungen und Alkoholen bei Siedetemperatur des Reaktionsgemisches, wobei das am niedrigsten siedende Edukt zunächst in unterstöchiometrischer Menge eingesetzt wird, das entstehende Dampfgemisch aus vorwiegend Wasser und der am niedrigsten siedenden Komponente an einer Membran entwässert wird, das entwässerte Dampfgemisch in das Reaktionsgemisch zurückgeführt und das am niedrigsten siedende Edukt im Laufe der Reaktion dem Reaktionsgemisch nachgesetzt wird.

Es ist bereits bekannt, bei chemischen Gleichgewichtsreaktionen, in denen Wasser gebildet wird, dies mit Hilfe von semipermeablen Membranen durch Pervaporation oder Dampfpermeation abzutrennen, hierdurch das Gleichgewicht zu verschieben und die Reaktion so zu vervollständigen. Solche Membranverfahren zur Fraktionierung von Gemischen mit organischen Komponenten sind in der Literatur ausführlich beschrieben (Rautenbach, Chem. Ing. Techn. 61 (1989) S. 539 - 544). Pervaporation und Dampfpermeation wurden ebenfalls schon ausführlich beschrieben (DE 3 610 011, DE-A 4 019 170 und EP-A 294 827).

Generell wird bei diesen Verfahren das zu trennende Stoffgemisch (Feed) an einer Membran entlanggeführt, die für die einzelnen Verbindungen des zulaufenden Stoffgemisches unterschiedliche Permeabilitäten aufweist. Triebkraft für den Stofftransport durch die Membran ist dabei eine transmembrane Differenz des elektrochemischen Potentials der einzelnen Stoffe des Zulaufgemisches. Im Falle der Pervaporation und Dampfpermeation wird diese Potentialdifferenz durch ein an der dem Feed abgewandten Seite der Membran (Permeatseite) angelegtes Vakuum oder durch Spülen der Permeatseite mit Inertgas aufgeprägt, was ein Ausschleusen der bevorzugt permeierenden Komponenten aus dem Feed zur Folge hat. An die Feedseite der Membran wird im allgemeinen ein erhöhter Druck angelegt. Das abgereicherte Feed wird als Retentat und die auf die Permeatseite übergehenden Stoffmengen werden als Permeat bezeichnet. Das Trennverhalten von Membranen ist stark temperaturabhängig, wird aber durch die thermische Beständigkeit der eingesetzten Membran begrenzt. So wird beispielsweise die Temperaturbelastbarkeit der Membran Pervap® 1000 der Firma Deutsche Carbone, GFT, vom Typ Polyvinylalkohol/Polyacrylnitril mit 100°C angegeben.

Die Pervaporation wird u.a. von Klatt (Dissertationsschrift "Zum Einsatz der Pervaporation im Umfeld der chemischen Industrie", Rheinisch-Westfälische Technische Hochschule (RWTH) Aachen 1993) beschriebenen. Die Pervaporation zeichnet sich dadurch aus, dass das Feedgemisch in flüssiger Form knapp unterhalb des Siedepunktes bei gegebenem Systemdruck zugeführt wird. Der Systemdruck muss dabei so gewählt werden, dass die Temperatur des zu trennenden Stoffgemisches an der Membran möglichst der optimalen Temperatur hinsichtlich Permeatfluss und Selektivität entspricht, keinesfalls aber oberhalb der maximal zulässigen Betriebstemperatur der Membran liegen darf (Membranbeständigkeit). Die Dampfpermeation wird u.a. von Helmus (Dissertationsschrift "Dampfpermeation - Trennvermögen, Prozessentwicklung und Einsatzmöglichkeiten", RWTH Aachen 1994) ausführlich beschrieben. Bei der Dampfpermeation wird das Feed im Gegensatz zur Pervaporation dampfförmig über die Membran geführt.

Kitha berichtet in Chemistry Letters (Chem. Soc. of Japan) 1987, 2053-2056 über die Veresterung von Carbonsäuren mit Alkoholen, zum Beispiel mit Ethanol, wobei aus dem vorgelegten Reaktionsgemisch Ethanol/Wasser entsprechend dem thermodynamischen Gleichgewicht abgedampft, der entweichende Dampf entlang einer Dampfpermeationsmembran geführt, und das an Wasser abgereicherte Retentat in das Reaktionsgemisch zurückgeführt wird. Mit dem beschriebenen Verfahren werden die erreichbaren Umsetzungsgrade bis zur vollständigen Umsetzung der Carbonsäure angehoben. Als Membranen werden Polyimid-, Chitosan- und Nafion® -Membranen eingesetzt, wobei insbesondere die eingesetzten Polyimidmembranen ausreichende Selektivitäten aufweisen. Gref(Proc. Fourth Int. Conf. Pervaporation, Proc. Chem. Ind. 1989, 344) beschreibt ebenfalls den Einsatz der Pervaporation, zur Auskreisung von Reaktionswasser bei der Herstellung von Carbonsäureestem unter Einsatz von PVA-Membranen. Sander (EP 299 577) beschreibt den Einsatz einer PVA-Membran zur selektiven Abtrennung von Wasser aus dem Dampf eines Reaktionsgemisches bei der Herstellung von Alkoholaten aus NaOH und Alkohol. Blum (DE 4 019 170 A1) berichtet über den erfolgreichen Einsatz der Dampfpermeation zur Abtrennung von Wasser bei der Herstellung von Dodecylacetat und Isopropylmyristat (Veresterungsreaktionen von Alkoholen mit Carbonsäuren) in einem Blasenreaktor. In EP 0 476 370 wird der Einsatz der Pervaporation/Dampfpermeation zur Abtrennung von Wasser aus einem Wasser und Alkohole und/oder Carbonsäuren und/ oder Carbonsäureester enthaltenden Gemisch beschrieben, wobei eine Membran verwendet wird, die durch Plasmapolymerisation erhalten worden ist. In EP-A 691 324 wird der Einsatz der Dampfpermeation zur Herstellung von Maleinsäurealkylestern aus Maleinsäureanhydrid und Alkohol beschrieben, wobei das dem Modul zugeführte Dampfgemisch kondensierte Anteile enthält, um eine Überhitzung des Dampfgemisches beim Überströmen der Membran und der damit verbundenen Leistungsminderung der Membran zu vermeiden.

Allen beschriebenen Verfahrensweisen ist gemeinsam, daß das am niedrigsten siedende Edukt (in vielen Fällen der Alkohol) mindestens stöchiometrisch, in der Regel aber überstöchiometrisch zugegeben wird. Hierdurch wird angestrebt, das Reaktionsgleichgewicht zu höheren Umsetzungsgraden der eingesetzten Säure oder das eingesetzten Alkalimetallhydroxids zu verschieben, um auf diese Weise gleichzeitig die Reaktionsdauer zu verkürzen. Kitha (s.o.) setzt in seinen Untersuchungen zur Veresterung von Ölsäure mit Ethanol einen stöchiometrischen Überschuß an Ethanol von 2:1 und 3:1 ein. Sander (s.o.) gibt für die Herstellung von Alkoholaten aus Alkalihydroxid und Alkohol unter Auskreisung des Reaktionswassers mit Dampfpermeation einen Alkoholüberschuß von 1:2 bis 1:10 an (Seite 2 Spalte 2 Zeile 20). Gref (s.o.) hat den Einfluß des Überschußfaktors bei der Veresterung von Propionsäure mit Isopropanol bei Einsatz der Pervaporation zum Auskreisen des Reaktionswassers systematisch untersucht und kommt zu dem Schluß, daß ein Herabsetzen des Überschußfaktors eines Eduktes zu längeren Reaktionszeiten hinsichtlich des Umsatzes der Minderkomponente führt (dortiges Bild 13). Als Optimum gibt Gref den stöchiometrischen Einsatz der Edukte an. Blum (s.o.) weist ebenfalls auf ein Optimum bei stöchiometrischen Einsatz der Edukte und Einsatz der Dampfpermeation zum Auskreisen des Reaktionswassers hin.

Überraschenderweise wurde nun gefunden, daß die Reaktionszeiten bei Veresterungsreaktionen und bei der Bildung von Alkalimetallalkoholaten aus wäßrigen Alkalimetallhydroxid-Lösungen und Alkoholen unter Einsatz der Dampfpermeation bzw. Pervaporation deutlich reduziert werden können, wenn man das am niedrigsten siedende Edukt (in vielen Fällen den Alkohol) zu Beginn der Reaktion zusammen mit dem jeweils anderen Edukt unterstöchiometrisch vorlegt, gegebenenfalls im Falle einer Veresterungsreaktion in Gegenwart eines Katalysators das Reaktionsgemisch zum Sieden bringt, das aus dem siedenden Reaktionsgemisch gebildete und entweichende Dampfgemisch als Feed an einer Membran, an der Wasser ausgeschleust wird, entlangführt, das so entwässerte Dampfgemisch (Retentat) in das Reaktionsgemisch zurückführt und im Laufe der Reaktion zur vollständigen Umsetzung das unterstöchiometrisch vorgelegte Edukt nachsetzt.

Die Erfindung betrifft demnach ein Verfahren zur Herstellung von Estern oder Alkoholaten unter Abtrennung von Wasser aus Reaktionsgemischen von Säuren bzw. Säureanhydriden oder von wässrigen Alkalimetallhydroxid-Lösungen mit Alkoholen unter Einsatz der Dampfpermeation/Pervaporation bei Siedetemperatur des Reaktionsgemisches, das dadurch gekennzeichnet ist, dass das am niedrigsten siedende Edukt gemeinsam mit dem jeweils anderen Edukt und im Falle von Veresterungen mit oder ohne Veresterungskatalysator als Reaktionsgemisch vorgelegt wird, wobei das am niedrigsten siedende Edukt in unterstöchiometrischer Menge, bezogen auf das jeweils andere Edukt, eingesetzt wird, das Reaktionsgemisch zum Sieden erhitzt wird und das aus dem siedenden Reaktionsgemisch gebildete Dampfgemisch, gegebenenfalls mit Anteilen an aus dem Dampfgemisch gebildeter kondensierter Phase, als Feed an einer Membran, an der Wasser abgetrennt wird, entlanggeführt wird, das an Wasser abgereicherte Dampfgemisch in das Reaktionsgemisch zurückgeführt wird und während der Reaktion das am niedrigsten siedende Edukt dem Reaktionsgemisch nachgesetzt wird.

Das erfindungsgemäße Verfahren ist sowohl im Falle der Veresterung als auch im Falle der Alkoholatbildung durch die Gemeinsamkeiten gekennzeichnet, dass zwei Edukte - im Falle der Veresterung eine Säure oder ein Säureanhydrid und ein Alkohol und im Falle der Alkoholatbildung eine wässrige Alkalimetallhydroxid-Lösung und ein Alkohol - miteinander unter Wasseraustritt reagieren, dass diese Reaktionen in Gegenwart von Wasser in umgekehrter Richtung unter Rückbildung der Edukte ablaufen (anstelle des Edukts Säureanhydrid wird jedoch die korrespondierende Säure zurückgebildet) und somit eine Gleichgewichtsreaktion darstellen und dass schließlich durch die Ausschleusung des Wassers aus dem Reaktionsgemisch die Reaktion unter Verbrauch der Edukte fortschreitet und zur Vollendung kommt.

Das erfindungsgemäß auszuschleusende Wasser ist in erster Linie das Reaktionswasser, beispielsweise aus den Gleichungen: 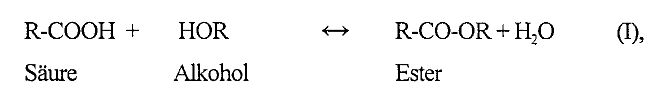 bzw. 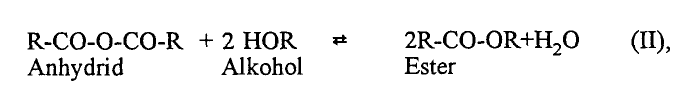 bzw.

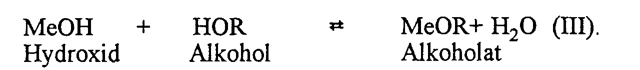

Selbstverständlich kommen als Säuren auch anorganische Säuren und als Anhydride auch cyclische Anhydride in Frage. Den Alkoholen als organische Hydroxyverbindung sind bei der Veresterung die Phenole äquivalent und erfindungsgemäß ausdrücklich einbezogen.

Neben dem Reaktionswasser kann jedoch im erfindungsgemäßen Verfahren auch Wasser anderer Herkunft aus dem Reaktionsgemisch ausgeschleust werden. Solches Wasser ist beispielsweise Verdünnungswasser des Edukts Alkalimetallhydroxid-Lösung oder solches der Edukte Säure oder Alkohol in verdünnter Form, so etwa der Einsatz von azeotrop siedendem 96 %igem Ethanol oder anderen Edukt/Wasser-Azeotropen, bei denen eine vorherige separate Entwässerung entfallen kann, sowie schließlich Kristallwasser, etwa bei der Oxalsäure (COOH)₂ · 2H₂O. Solches Wasser und Wasser noch weiterer Herkunft ist dem Fachmann bekannt.

Als Alkoholkomponente können im erfindungsgemäßen Verfahren geradkettige oder verzweigte, offenkettige oder cyclische, gesättigte oder ungesättigte C₁-C₈-Alkohole eingesetzt werden, wie Methanol, Ethanol, n-Propanol, i-Propanol, n-Butanol, i-Butanol, die isomeren Pentanole, Hexanole, Heptanole und Octanole. Besonders bevorzugt sind solche Alkohole, die mit Wasser ein Azeotrop bilden und dadurch die destillative Wasserabtrennung für die Rückführung der Alkoholkomponente unmöglich machen, wie z.B. Ethanol, n-Propanol und iso-Propanol. Weiterhin einsetzbar sind Phenol und substituierte Phenole, wie Kresol oder Chlorbenzol, die wie die Alkohole veresterbare organische Hydroxyverbindungen sind.

Als zu veresternde Säuren seien für das erfindungsgemäße Verfahren unter Einbeziehung des Carboxyl-C-Atoms genannt: aliphatische gesättigte oder ungesättigte, geradkettige oder verzweigte C₁-C₂₀-Carbonsäuren, wie Ameisensäure, Essigsäure, Propionsäure, Buttersäure, Valeriansäure, Capronsäure, Caprylsäure, Caprinsäure, Laurinsäure, Palmitinsäure, Stearinsäure, Acrylsäure, Methacrylsäure, Crotonsäure, Vinylessigsäure, Ölsäure, Elaidinsäure, Linolsäure, Linolensäure und Anhydride dieser Säuren; Halogenderivate solcher Carbonsäuren und ihrer Anhydride; schwefelhaltige Derivate von Carbonsäuren, wie Thiodiglykolsäure; aliphatische gesättigte oder ungesättigte C₂-C₁₂-Dicarbonsäuren, wie Oxalsäure, Malonsäure, Bemsteinsäure, Glutarsäure, Adipinsäure, Suberinsäure, Maleinsäure, Fumarsäure, deren Monomethyl- und Monohalogen-Derivate und Anhydride; cycloaliphatische C₄-C₁₂-Carbonsäuren, wie Cyclopropancarbonsäure, Monomethyl- und Dimethyl-cyclopropancarbonsäure, Cyclobutancarbonsäure, Cyclopentancarbonsäure, Cyclohexancarbonsäure, Monomethyl-, Dimethyl- und Trimethyl-cyclohexancarbonsäure; aromatische Mono- und Dicarbonsäuren, wie Benzoesäure, Phthalsäure, Terephthalsäure, Nitro-, Chlor-, Methyl- und Hydroxybenzoesäure und deren Anhydride; anorganische Säuren, wie Schwefelsäure, Phosphorsäure, Borsäure.

Die gemeinsame Vorlage von Alkohol, Säure und gegebenenfalls Veresterungskatalysator im Falle einer Verestereung bzw. die gemeinsame Vorlage von Alkohol und Alkalimetallhydroxid-Lösung im Falle einer Alkoholbildung bedeutet, dass diese Stoffe vor Eintritt des Siedevorgangs im Reaktionsgemisch sind. Jedoch liegt das am niedrigsten siedende Edukt zunächst in stöchiometrischem Unterschuss vor. Im allgemeinen wird zu Beginn der Reaktion das am niedrigsten siedende Edukt zu 10 % bis unterhalb 100 % der Stöchiometrie und besonders bevorzugt 30 % bis 80 % vorgelegt, wobei sich die Stöchiometrie auf die Umsetzungen gemäß obigen Formeln (I), (II) oder (III) bezieht.

Eine völlig analoge Verfahrensweise ist möglich, wenn bei einer Veresterungsreaktion zwischen einer niedrig siedenden Carbonsäure und einem Alkohol oder Phenol mit einem Siedepunkt oberhalb dem der Carbonsäure das Reaktionswasser gemeinsam mit der Carbonsäure verdampft und an der Membran entlanggeführt wird. In diesem Fall wird die Carbonsäure zunächst unterstöchiometrisch vorgelegt und während der Reaktion nachgesetzt.

Eine weiterhin völlig analoge Verfahrensweise ist möglich, wenn aus wässriger Alkalimetallhydroxid-Lösung und C₁-C₈-Alkanol eine wasserarme oder wasserfreie Alkalimetallalkanolat-Lösung in dem betreffenden Alkanol hergestellt werden soll. Auch hier wird die niedriger siedende Komponente Alkanol (dies gilt wegen der Azeotropbildung auch für oberhalb 100°C siedende Alkanole) zunächst unterstöchiometrisch vorgelegt und während der Reaktion nachgesetzt

Die erfindungsgemäße Verfahrensweise schließt nicht aus, dass die zunächst nur unterstöchiometrisch vorgelegte Reaktionskomponente am Ende der Reaktion und nach absatzweisem oder kontinuierlichem Nachsetzen schließlich in einem Überschuss vorliegt, sei es, dass die jeweils andere Komponte wertvoller ist und vollständig umgesetzt werden soll, sei es, dass die überschüssig vorliegende Komponente als Lösungsmittel dienen soll.

Allgemein wird das Reaktionswasser gemeinsam mit der am leichtesten siedenden Komponente aus dem Reaktionsgemisch verdampft und an der Membran entlanggeführt. Dies kann im Falle niedrig siedender Ester dieser niedrig siedende Ester sein. In jedem Falle aber wird erfindungsgemäß das am niedrigsten siedende Edukt im Unterschuss vorgelegt und erst während der Reaktion nachgesetzt.

Die erfindungsgemäß verwendeten Membranen können beispielsweise aus Cellulosediacetat, Polyimid, Cellulosetriacetat, Polyethersulfon, Polyamid oder Polyvinylalkohol hergestellt werden oder eine durch Plasmapolymerisation hergestellte porenfreie Schicht darstellen. Die Polymermaterialien haben hierbei im allgemeinen ein Molekulargewicht zwischen 15 000 und 200 000. Polyvinylalkohol wird im allgemeinen durch weitgehende Verseifung von Polyvinylacetat hergestellt; die Verseifungsgrade sollen bevorzugt über 95 %, besonders bevorzugt über 98 % liegen. Wegen der Wasserlöslichkeit von Polyvinylalkohol wird dies im allgemeinen in vernetzter Form eingesetzt. Eine solche Vernetzung kann in einer Veretherung, Veresterung oder Acetalisierung mit mehrfunktionellen Verbindungen bestehen. Solche Membranen sind dem Fachmann bekannt und sind beispielsweise in EP-592 883 beschrieben. Jedoch können neben den genannten organischen Membranen mit gleichem Erfolg auch anorganische Membranen, wie keramische Membranen oder Zeolith-Membranen, eingesetzt werden.

In bevorzugter Form werden Compositmembranen eingesetzt, die im allgemeinen aus mehreren Schichten, nämlich einer Trägerschicht, einer porösen Schicht und der eigentlichen Trennschicht bestehen. Als Trägerschicht kommen im allgemeinen hochporöse flexible Gewebe oder Vliese aus Fasern, einschließlich Metallfasern, Polyolefinen, Polysulfonen, Polyetherimiden, Polyphenylsulfiden oder Kohlenstoff in Frage; gleichfalls geeignet sind poröse Strukturen aus Glas, Keramik, Graphit oder Metallen. Die poröse Stützschicht hat bevorzugt eine asymmetrische Porenstruktur. Solche porösen Stützschichten können beispielsweise aus Polysulfon, Polyethersulfon, Polyetherimid, Polyvinylidenfluorid, hydrolysiertem Cellulosetriacetat, Polyphenylensulfid, Polyacrylnitril, Polyester, Polytetrafluorethylen, Polyethylen, Polyvinylalkohol, Copolymeren von perfluorierten Polyolefinen sowie anderen geeigneten Polymerisaten hergestellt werden. Die Molekulargewichte können gleichfalls im Bereich von 15 000 bis 200 000 liegen. Die eigentliche Trennschicht kann wiederum aus Cellulosediacetat, Cellulosetriacetat, Polyvinylalkohol oder durch Plasmapolymerisation hergestellten Schicht bestehen. Polyvinylalkohol wird in der oben beschriebenen Weise vernetzt, um dem Angriff von Wasser bei höheren Temperaturen besser zu widerstehen. Die Membranen können als Wickelmodul, Plattenmodul, Kissenmodul, Hohlfaser- oder Kapillarmodul eingesetzt werden. Besonders bevorzugt sind Wickelmodule.

Die Temperaturen an der Membran richten sich naturgemäß vorwiegend nach der Beständigkeit der eingesetzten Membran. In der Regel wird bei Temperaturen von 50 bis 150°C, bevorzugt bei 70 bis 130°C, besonders bevorzugt bei der Siedetemperatur des Reaktionsgemisches gearbeitet. Die Siedetemperatur des Reaktionsgemisches ist abhängig von seiner stofflichen Zusammensetzung; dies ist dem Fachmann geläufig. Diese Temperaturen können jedoch durch Arbeiten unter Druck oder Vakuum erhöht bzw. erniedrigt werden. Erhöhte Temperaturen und damit einhergehende Druckerhöhungen können sich beispielsweise dann als vorteilhaft erweisen, wenn das Reaktionsgemisch bei Normaldruck eine Temperatur besitzt, die unterhalb der optimalen Arbeitstemperatur der verwendeten Membran liegt.

Eine bevorzugte Betriebsweise ist, den Siedepunkt des Reaktionsgemisches über den Anteil an Alkohol zu Beginn und den im Verlauf der Reaktion nachgesetzten Alkohol so einzustellen, daß die Temperatur des aus dem Reaktionsgemisch entstehenden Dampfes die maximal zulässige Betriebstemperatur der eingesetzten Membran nicht überschreitet, gegebenenfalls muß zusätzlich der Betriebsdruck entsprechend eingestellt werden. In besonders bevorzugter Weise wird die Temperatur des Dampfgemisches, mit der es das Membranmodul anströmt, so eingestellt, daß die maximale Betriebstemperatur der eingesetzten Membran nicht überschritten wird. Diese Temperatur kann verschieden von der sein, mit der das Dampfgemisch dem Reaktionsgemisch entweicht.

Das Verfahren wird in einer bevorzugten Ausführungsform so durchgeführt, daß das Feed dem Membranmodul als Gemisch aus Dampf und kondensierter Phase zugeführt wird. Dabei wird ein Teil des Dampfes, vorzugsweise in einem dem Modul vorgeschalteten Wärmetauscher kondensiert, so daß das Feed einen Anteil an kondensierter Phase von 5-90 Gew.-% besitzt, vorzugsweise 5-50 Gew.-%, insbesondere 10-20 Gew.-%.

Eine besonders vorteilhafte Entwässerung des Feeds kann erreicht werden, wenn der als Retentat aus dem Membranmodul austretende Feed-Dampf im Sattdampfzustand ist.

Die vorzugsweise kontinuierlich verlaufende Rückführung des entwässerten Retentats in die Reaktionsmischung kann entweder direkt als Dampf eingeblasen werden oder in Form der, gegebenenfalls durch zwischengeschaltete Destillationskolonnen erhaltenen, kondensierten Phase erfolgen. Bevorzugt ist eine dampfförmige Rückführung.

Als gegebenenfalls im Falle einer Veresterung einzusetzender saurer Katalysator können sowohl anorganische wie organische Säuren verwendet werden, beispielsweise Schwefelsäure oder p-Toluolsulfonsäure. Die möglichen Mengen des Katalysators bewegen sich dabei in der für Veresterungsreaktionen üblichen Bereichen. In vielen Fällen reicht die Säurestärke der zu veresternden Säure zur katalytischen Wirkung aus.

Das Verfahren kann im übrigen diskontinuierlich oder kontinuierlich durchgeführt werden, wobei die kontinuierliche Fahrweise in einem Rührkessel mit Überlauf oder bevorzugt einer Reaktionskolonne durchgeführt werden kann.

Das erfindungsgemäße Verfahren wird in einer bevorzugten Ausführungsform so durchgeführt, daß in einem Reaktionskessel das am niedrigsten siedende Edukt, gegebenenfalls im Falle einer Veresterung mit einem Katalysator, unterstöchiometrisch vorgelegt, die zu veresternde Säure bzw. die Alkalimetalhydroxid-Lösung zugegeben und auf Siedetemperatur aufgeheizt wird. Das aus dem Reaktionsgemisch entstehende Dampfgemisch wird direkt oder über eine Destillationskolonne dem Membranmodul dampfförmig mit Anteilen an aus dem Dampf gebildeten Kondensates zugeführt. Das wasserreiche Permeat wird abgezogen und das Retentat ständig während der Reaktion in die Reaktionsmischung zurückgeführt. Im Laufe der Reaktion wird dem Reaktionsgemisch zusätzlich das am niedrigsten siedende Edukt, nachgesetzt.

Nach beendeter Reaktion werden im Falle einer Veresterung gegebenenfalls die sauren Bestandteile des Reaktionsansatzes (Katalysator, nicht umgesetzte Säure) mit einer Base neutralisiert und der Ester destillativ isoliert. Nach diesem Verfahren werden Ester in Ausbeuten von über 95% und in hohen Reinheiten (größer als 99 %) dargestellt.

Im Falle der Alkoholatbildung liegt nach der Ausschleusung des Wassers (Reaktionswasser und Lösungswasser der Alkalimetallhydroxid-Lösung) eine gebrauchsfertige Alkoholat-Lösung vor, die in der Regel Alkohol in einer über die Reaktionsstöchiometrie hinausgehenden Menge als Lösungsmittel enthält.

Die bisher in der Literatur beschriebenen Verfahren zum Einsatz der Dampfpermeation/Pervaporation zum Auskreisen von Reaktionswasser gehen im Falle einer Veresterung von einem überstöchiometrischen Vorlegen des Alkohols aus, auch wenn der Alkohol das am niedrigsten siedende Edukt ist, um eine ausreichende Produktausbeute und kurze Reaktionszeiten zu erreichen. Für einen optimalen Betrieb bei Einsatz der Dampfpermeation/Pervaporation wird ein mindestens stöchiometrischer Einsatz empfohlen. Durch eine unterstöchiometrische Vorlage des am niedrigsten siedenden Edukts und Nachsetzen des am niedrigsten siedenden Edukts im Laufe der Reaktion wird im erfindungsgemäßen Verfahren die Reaktionszeit bei gleichen Umsetzungsgraden überraschenderweise deutlich reduziert. Dies trifft in völlig analoger Weise auf die Alkoholatbildung zu. Die unterstöchiometrische Vorlage des am niedrigsten siedenden Edukts bei dem beschriebenen Verfahren stellt die Frage nach der Verringerung der Reaktionsgeschwindigkeit, die aufgrund der unterstöchiometrischen Vorlage des am niedrigsten siedenden Edukts für den Fachmann zu erwarten war und die somit zu einer Verlängerung der Verweilzeit im Apparat führen sollte, was letztlich wirtschaftlich nicht mehr zu vertreten wäre. Das erfindungsgemäße Verfahren führt somit auf eine einfach durchzuführende Weise zu einer deutlichen Reduzierung der erforderlichen Reaktionszeiten.

Das erfindungsgemäße Verfahren sei an folgenden Beispielen demonstriert.

### Beispiele

### Beispiel 1

In einem 2-1-Rührbehälter wurden 152 g Ethanol (ca. 20 %-iger Unterschuss) und 5 g p-Toluolsulfonsäure als Katalysator vorgelegt und das Gemisch auf 50°C erwärmt. Anschließend wurden 202 g Maleinsäureanhydrid zugesetzt und das Gemisch bei Umgebungsdruck zum Sieden gebracht. Das entstehende Dampfgemisch wurde einer Dampfpermeationstestzelle mit einer Membranfläche von 0,02 m² zugeführt und anschließend in das Reaktionsgemisch zurückgeführt. Das Retentat enthielt einen Anteil an aus dem Dampf gebildeten Kondensat von ca. 10 Gew.-%. Als Membran wurde der Typ CM® -CE-01 der Firma Cm-Celfa eingesetzt. Das Permeat wurde bei einem Permeatdruck von 10 mbar kondensiert und durch eine Vakuumwechselvorlage aus dem Vakuumbereich ausgetragen. Dem Reaktionsgemisch wurde im Laufe der Reaktion weiteres Ethanol in der Weise nachgesetzt, dass die Siedetemperatur konstant gehalten wurde und die Zulauftemperatur der Testzelle 100°C nicht überstieg. Die gesamte nachgesetzte Ethanolmenge betrug 135 g. Bei einer Reaktonszeit von 7 Stunden wurde ein Umsetzungsgrad von 98 % erreicht.

### Vergleichsbeispiel 1.1

In einem 2-1-Rührbehälter wurden 288 g Ethanol (ca. 50%iger Überschuss) und 5 g p-Toluolsulfonsäure als Katalysator vorgelegt und das Gemisch auf 50°C erwärmt. Anschließend wurden 202 g Maleinsäureanhydrid zugesetzt und das Gemisch bei Umgebungsdruck zum Sieden gebracht. Das entstehende Dampfgemisch wurde einer Dampfpermeationstestzelle mit einer Membranfläche von 0,02 m² zugeführt und anschließend das Retentat in das Reaktionsgemisch zurückgeführt. Das Retentat enthielt Anteile an aus dem Dampf gebildeten Kondensat von ca. 10 Gew.%. Als Membran wurde der Typ CM® -CE-01 der Firma Cm-Celfa eingesetzt. Das Permeat wurde bei einem Permeatdruck von 10 mbar kondensiert und durch eine Vakuumwechselvorlage aus dem Vakuumbereich ausgetragen. Bei einer Reaktonszeit von 10 Stunden wurde ein Umsetzungsgrad von 98 % erreicht.

### Beispiel 2

In einem 2-l-Rührbehälter wurden 191 g Ethanol (ca. 20%iger Unterschuss) und 7g p-Toluolsulfonsäure als Katalysator vorgelegt und das Gemisch auf 50°C erwärmt. Anschließend wurden 334 g Oxalsäuredihydrat zugesetzt und das Gemisch bei Umgebungsdruck zum Sieden gebracht. Das entstehende Dampfgemisch wurde einer Dampfpermeationstestzelle mit einer Membranfläche von 0,02 m² zugeführt und anschließend in das Reaktionsgemisch zurückgeführt. Das Retentat enthielt Anteile an aus dem Dampf gebildeten Kondensat von ca. 10 Gew.-%. Als Membran wurde der Typ CM® -CE-01 der Firma Cm-Celfa eingesetzt. Das Permeat wurde bei einem Permeatdruck von 10 mbar kondensiert und durch eine Vakuumwechselvorlage aus dem Vakuumbereich ausgetragen. Dem Reaktionsgemisch wurde im Laufe der Reaktion weiteres Ethanol nachgesetzt, und zwar so, dass die Siedetemperatur konstant gehalten wurde und die Zulauftemperatur der Testzelle 100°C nicht überschritt. Die gesamte nachgesetzte Ethanolmenge betrug 168 g. Bei einer Reaktonszeit von 15 Stunden wurde ein Umsetzungsgrad von 98 % erreicht.

### Vergleichsbeispiel 2.1

In einem 2-1-Rührbehälter wurden 359 g Ethanol (ca. 50%-iger Überschuss) und 7 g p-Toluolsulfonsäure als Katalysator vorgelegt und das Gemisch auf 50°C erwärmt. Anschließend wurden 334 g Oxalsäuredihydrat zugesetzt und das Gemisch bei Umgebungsdruck zum Sieden gebracht. Das entstehende Dampfgemisch wurde einer Dampfpermeationstestzelle mit einer Membranfläche von 0,02 m² zugeführt und anschließend in das Reaktionsgemisch zurückgeführt. Das Retentat enthielt Anteile an aus dem Dampf gebildeten Kondensat von ca. 10 Gew.-%. Als Membran wurde der Typ CM® -CE-01 der Firma Cm-Celfa eingesetzt. Das Permeat wurde bei einem Permeatdruck von 10 mbar kondensiert und durch eine Vakuumwechselvorlage aus dem Vakuumbereich ausgetragen. Erst nach einer Reaktionszeit von 25 Stunden wurde ein Umsetzungsgrad von 98 % erreicht.

### Vergleichsbeispiel 2.2

In einem 2-1-Rührbehälter wurden 239 g Ethanol (stöchiometrischer Einsatz) und 7g p-Toluolsulfonsäure als Katalysator vorgelegt und das Gemisch auf 50°C erwärmt. Anschließend wurden 334 g Oxalsäuredihydrat zugesetzt und das Gemisch bei Umgebungsdruck zum Sieden gebracht. Das entstehende Dampfgemisch wurde einer Dampfpermeationstestzelle mit einer Membranfläche von 0,02 m² zugeführt und anschließend in das Reaktionsgemisch zurückgeführt. Das Retentat enthielt Anteile an aus dem Dampf gebildeten Kondensat von ca. 10 Gew.-%. Als Membran wurde der Typ CM® -CE-01 der Firma Cm-Celfa eingesetzt. Das Permeat wurde bei einem Permeatdruck von 10 mbar kondensiert und durch eine Vakuumwechselvorlage aus dem Vakuumbereich ausgetragen. Bei einer Reaktionszeit von 25 Stunden wurde ein Umsetzungsgrad von 92 % erreicht.

## Patentansprüche

1. Verfahren zur Herstellung von Estern oder Alkoholaten unter Abtrennung von Wasser aus Reaktionsgemischen von Säuren bzw. Säureanhydriden oder von wässrigen Alkalimetallhydroxid-Lösungen mit Alkoholen unter Einsatz der Dampfpermeation/Pervaporation bei Siedetemperatur des Reaktionsgemisches, dadurch gekennzeichnet, dass das am niedrigsten siedende Edukt gemeinsam mit dem jeweils anderen Edukt und im Falle von Veresterungen mit oder ohne Veresterungskatalysator als Reaktionsgemisch vorgelegt wird, wobei das am niedrigsten siedende Edukt in unterstöchiometrischer Menge, bezogen auf das jeweils andere Edukt, eingesetzt wird, das Reaktionsgemisch zum Sieden erhitzt wird und das aus dem siedenden Reaktionsgemisch gebildete Dampfgemisch, gegebenenfalls mit Anteilen an aus dem Dampfgemisch gebildeter kondensierter Phase, als Feed an einer Membran, an der Wasser abgetrennt wird, entlanggeführt wird, das an Wasser abgereicherte Dampfgemisch in das Reaktionsgemisch zurückgeführt wird und während der Reaktion das am niedrigsten siedende Edukt dem Reaktionsgemisch nachgesetzt wird.

2. Verfahren nach Anspruch 1, dadurch gekennzeichnet, dass als C₁-C₈-Alkohole solche eingesetzt werden, die mit Wasser ein Azeotrop bilden.

3. Verfahren nach Anspruch 1, dadurch gekennzeichnet, dass die aktive Schicht der verwendeten Membran aus Cellulosediacetat, Cellulosetriacetat, Polyvinylalkohol, Polyimid, Polyethersulfon oder Polyamid hergestellt worden ist oder eine durch Plasmapolymerisation hergestellte porenfreie Schicht, eine keramische Membran oder eine Zeolith-Membran darstellt.

4. Verfahren nach Anspruch 1, dadurch gekennzeichnet, dass als Membran eine Composit-Membran eingesetzt wird.

5. Verfahren nach Anspruch 1, dadurch gekennzeichnet, dass der Anteil der kondensierten Phase im Feed für die Membran 5 bis 90 Gew.-%, vorzugsweise 5 bis 50 Gew.-% insbesondere 10 bis 20 Gew.-% beträgt.

6. Verfahren nach Anspruch 1, dadurch gekennzeichnet, dass das am niedrigsten siedende Edukt in einer Menge von 10% bis unterhalb 100% und besonders bevorzugt 30 bis 80% der stöchiometischen Menge mit dem anderen Edukt gemeinsam vorgelegt wird.

7. Verfahren nach Anspruch 6, dadurch gekennzeichnet, dass die Siedetemperatur des vorgelegten Gemisches die maximal zulässige Betriebstemperatur der eingesetzten Membran nicht überschreitet, bevorzugt die Dampftemperatur des aus dem flüssigen Reaktionsgemisch gebildeten Dampfes die maximal zulässige Betriebstemperatur der eingesetzten Membran nicht überschreitet, besonders bevorzugt das dem Membranmodul zuströmenden Dampfgemisch die maximale zulässige Betriebstemperatur der eingesetzten Membran nicht überschreitet.

8. Verfahren nach Anspruch 6, dadurch gekennzeichnet, dass das Nachsetzen des am niedrigsten siedenden Edukts derart geschieht, dass die maximal zulässige Betriebstemperatur der eingesetzten Membran während der gesamten Reaktion nicht überschritten, bevorzugt auf die maximal zulässige Betriebstemperatur der Membran gehalten wird.

## Claims

1. Process for the preparation of esters or alcoholates by removal of water from reaction mixtures of acids or acid anhydrides or of aqueous alkali metal hydroxide solutions with alcohols using vapour permeation/pervaporation at the boiling point of the reaction mixture, which comprises initially introducing, as the reaction mixture, the lowest-boiling reactant, together with the other particular reactant and, in the case of esterifications, with or without an esterification catalyst, the lowest-boiling reactant being employed in less than the stoichiometric amount, based on the other particular reactant, heating the reaction mixture to the boiling point and feeding the vapour mixture formed from the boiling reaction mixture, if appropriate together with contents of a condensed phase formed from the vapour mixture, as a feed along a membrane on which water is removed, recycling the vapour mixture depleted in water into the reaction mixture and topping up the reaction mixture with the lowest-boiling reactant during the reaction.

2. Process according to Claim 1, characterized in that the C₁-C₈-alcohols employed are those which form an azeotrope with water.

3. Process according to Claim 1, characterized in that the active layer of the membrane used has been prepared from cellulose diacetate, cellulose triacetate, polyvinyl alcohol, polyimide, polyether-sulphone or polyamide or is a pore-free layer prepared by plasma polymerization, a ceramic membrane or a zeolite membrane.

4. Process according to Claim 1, characterized in that a composite membrane is employed as the membrane.

5. Process according to Claim 1, characterized in that the content of condensed phase in the feed for the membrane is 5 to 90% by weight, preferably 5 to 50% by weight, in particular 10 to 20% by weight.

6. Process according to Claim 1, characterized in that the lowest-boiling reactant is initially introduced together with the other reactant in an amount of from 10% to below 100% and particularly preferably 30% to 80% of the stoichiometric amount.

7. Process according to Claim 6, characterized in that the boiling point of the mixture initially introduced does not exceed the maximum permitted operating temperature of the membrane employed, preferably the vapour temperature of the vapour formed from the liquid reaction mixture does not exceed the maximum permitted operating temperature of the membrane employed, and particularly preferably the vapour mixture flowing to the membrane module does not exceed the maximum permitted operating temperature of the membrane employed.

8. Process according to Claim 6, characterized in that the mixture is topped up with the lowest-boiling reactant such that the maximum permitted operating temperature of the membrane employed is not exceeded during the entire reaction, and the temperature is preferably kept at the maximum permitted operating temperature of the membrane.

## Revendications

1. Procédé pour la préparation d'esters ou d'alcoolates avec séparation d'eau à partir de mélanges de réaction consistant en acides ou anhydrides ou en solutions aqueuses d'hydroxydes de métaux alcalins et alcools avec exploitation de la perméation de vapeurs/pervaporation à la température d'ébullition du mélange de réaction, caractérisé en ce que l'on met en oeuvre en tant que mélange de réaction le composant de départ bouillant le plus bas avec l'autre composant de départ et, dans le cas d'une estérification, avec ou sans catalyseur d'estérification, le composant de départ bouillant le plus bas étant mis en oeuvre en défaut par rapport à l'autre composant de départ, on porte le mélange de réaction à l'ébullition et on envoie le mélange de vapeurs formé à partir du mélange de réaction bouillant, le cas échéant avec des fractions de la phase condensée formée à partir du mélange de vapeurs, en alimentation sur une membrane sur laquelle l'eau est séparée, on recycle le mélange de vapeurs appauvri en eau au mélange de réaction et, en cours de réaction, on ajoute au mélange de réaction des compléments du composant de départ bouillant le plus bas.

2. Procédé selon la revendication 1, caractérisé en ce que l'on met en oeuvre des alcools en C₁-C₈ qui forment des azéotropes avec l'eau.

3. Procédé selon la revendication 1, caractérisé en ce que la couche active de la membrane utilisée a été formée à partir de diacétate de cellulose, de triacétate de cellulose, d'alcool polyvinylique, d'un polyimide, d'une polyéthersulfone ou d'un polyamide ou consiste en une couche non poreuse préparée par polymérisation en plasma, une membrane céramique ou une membrane de zéolithe.

4. Procédé selon la revendication 1, caractérisé en ce que l'on utilise une membrane composite.

5. Procédé selon la revendication 1, caractérisé en ce que la proportion de la phase condensée dans l'alimentation à la membrane représente 5 à 90 % en poids, de préférence 5 à 50 % en poids, plus spécialement 10 à 20 % en poids.

6. Procédé selon la revendication 1, caractérisé en ce que l'on met en oeuvre le composant de départ bouillant le plus bas en quantité allant de 10 % jusqu'à moins de 100 % et de préférence de 30 à 80 % de la quantité théorique, avec l'autre composant de départ.

7. Procédé selon la revendication 6, caractérisé en ce que la température d'ébullition du mélange mis en oeuvre ne dépasse pas la température de fonctionnement maximale autorisée pour la membrane, de préférence la température des vapeurs formées à partir du mélange de réaction liquide ne dépasse pas la température de fonctionnement maximal autorisée pour la membrane et dans les meilleures conditions le mélange de vapeurs envoyé au module à membrane ne dépasse pas la température de fonctionnement maximale autorisée pour la membrane.

8. Procédé selon la revendication 6, caractérisé en ce que l'addition de compléments du composant de départ bouillant le plus bas est réalisée en sorte que pendant toute la réaction, on ne dépasse pas la température de fonctionnement maximale autorisée pour la membrane et de préférence on se tienne à la température de fonctionnement maximale autorisée pour la membrane.
